(19) Europäisches Patentamt
European Patent Office
Office européen des brevets

(11) Veröffentlichungsnummer: **0 192 148**
**B1**

# (12) EUROPÄISCHE PATENTSCHRIFT

(45) Veröffentlichungstag der Patentschrift: **18.01.89**

(51) Int. Cl.⁴: **C 07 D 277/32** // A01N43/80

(21) Anmeldenummer: **86101651.7**

(22) Anmeldetag: **10.02.86**

(54) **2,4-Dichlorthiazol-derivate und Verfahren zu ihrer Herstellung.**

(30) Priorität: **21.02.85 DE 3505900**

(43) Veröffentlichungstag der Anmeldung: **27.08.86 Patentblatt 86/35**

(45) Bekanntmachung des Hinweises auf die Patenterteilung: **18.01.89 Patentblatt 89/3**

(84) Benannte Vertragsstaaten: **CH DE FR GB IT LI**

(56) Entgegenhaltungen:
**EP-A- 0 053 765**
**EP-A- 0 115 811**
**DE-A- 2 844 270**
**DE-B- 2 213 865**

(73) Patentinhaber: **BAYER AG, Konzernverwaltung RP Patentabteilung, D-5090 Leverkusen 1 Bayerwerk (DE)**

(72) Erfinder: **Beck, Gunther, Dr., Am Mittelberg 19, D-5090 Leverkusen (DE)**
Erfinder: **Schubart, Rüdiger, Dr., An der Engelsfuhr 27, D-5060 Bergisch-Gladbach 2 (DE)**

EP 0 192 148 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2,4-Dichlorthiazol-Derivate und mehrere Verfahren zu ihrer Herstellung.

Es ist bereits bekannt, dass 2,4,5-Trichlorthiazol – herstellbar z.B. durch Umsetzung von Chloralformamid mit Thionylchlorid und Schwefel unter bestimmten Verfahrensbedingungen – insektizide Eigenschaften aufweist (vgl. EP-A-0 053 765).

Es wurden neue 2,4-Dichlorthiazol-Derivate der Formel

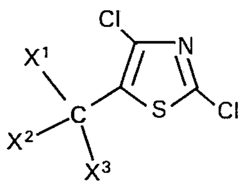
(I)

gefunden, in welcher

$X^1$ für Wasserstoff oder Chlor,
$X^2$ für Wasserstoff oder Chlor und
$X^3$ für Wasserstoff oder Chlor steht.

Im einzelnen handelt es sich um die folgenden Verbindungen

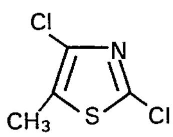
(Ia)

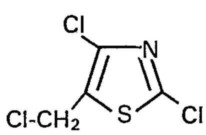
(Ib)

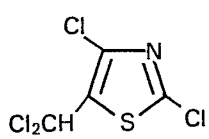
(Ic)

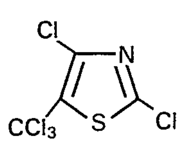
(Id)

Weiterhin wurde gefunden, dass man 2,4-Dichlorthiazol-Derivate der Formel (I) erhält, wenn man

a) 5-Methyl-2,4-thiazolidindion der Formel

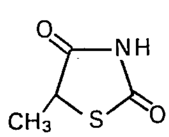
(II)

mit mindestens 2 Mol Phosphoroxidchlorid ($POCl_3$) in Gegenwart katalytischer Mengen N-Alkyl-substituierter Carbonsäureamide umsetzt, wobei man 2,4-Dichlor-5-methylthiazol der Formel (Ia) erhält,
oder wenn man

b) α) 2,4-Dichlor-5-methylthiazol der Formel (Ia),
β) 2,4-Dichlor-5-chlormethylthiazol der Formel (Ib) oder
γ) 2,4-Dichlor-5-dichlormethylthiazol der Formel (Ic)

bei erhöhter Temperatur unter Belichtung mit Chlor umsetzt.

Die erfindungsgemässen 2,4-Dichlorthiazol-Derivate sind durch die Formel (I) bzw. die Formeln (Ia) bis (Id) definiert.

Verwendet man bei dem erfindungsgemässen Verfahren (a) auf 1 Mol 5-Methyl-2,4-thiazolidindion 2 Mol Phosphoroxidchlorid, so kann der Verlauf der Umsetzung durch das folgende Formelschema wiedergegeben werden:

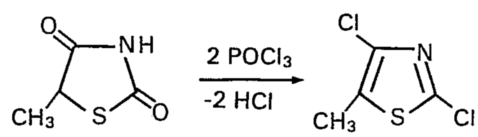


Verwendet man bei dem erfindungsgemässen Verfahren (b)/ Variante α) 2,4-Dichlor-5-methylthiazol als Ausgangsstoff, so kann der Verlauf der Umsetzung mit Chlor je nach Chlorierungsgrad durch die folgenden Formelschemata wiedergegeben werden:

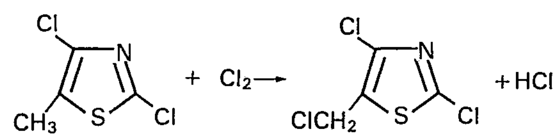


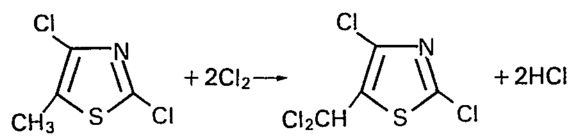


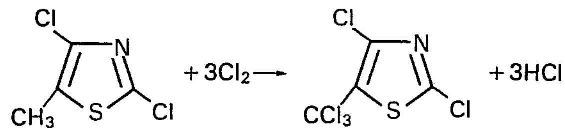


Verwendet man bei dem erfindungsgemässen Verfahren (b)/ Variante β) 2,4-Dichlor-5-chlormethylthiazol als Ausgangsstoff, so kann der Verlauf der Umsetzung mit Chlor je nach Chlorierungsgrad durch die folgenden Formelschemata wiedergegeben werden:

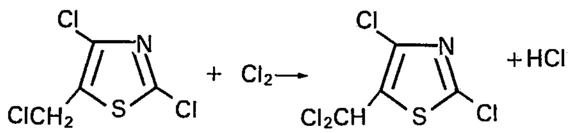

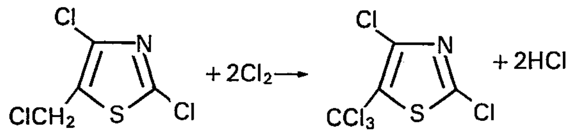

Verwendet man bei dem erfindungsgemässen Verfahren (b)/ Variante γ) 2,4-Dichlor-5-(dichlormethyl)-thiazol als Ausgangsstoff, so kann der Verlauf der Umsetzung mit Chlor durch das folgende Formelschema wiedergegeben werden:

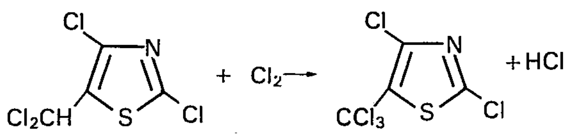

Das bei dem erfindungsgemässen Verfahren (a) als Ausgangsprodukt benötigte 5-Methyl-2,4-thiazolidindion ist bekannt.

Bei der Durchführung des erfindungsgemässen Verfahrens (a) sind für einen vollständigen Umsatz der Ausgangsverbindung 2 Mol Phosphoroxidchlorid erforderlich. Zur Erzielung einer besseren Ausbeute setzt man jedoch im allgemeinen einen Überschuss an Phosphoroxidchlorid ein, der bis zum fünffachen der stöchiometrisch erforderlichen Menge gehen kann, wobei es gleichzeitig als Lösungsmittel bzw. als Reaktionsmedium dient. Vorzugsweise arbeitet man mit 3 bis 8 Mol Phosphoroxidchlorid pro Mol 5-Methyl-2,4-thiazolidindion.

Als N-Alkyl-substituierte Carbonsäureamide seien genannt: Dimethylformamid, N,N-Dimethylacetamid, 1-Methyl-2-pyrrolidinon, Tetramethylharnstoff. Vorzugsweise arbeitet man mit Dimethylformamid. Im allgemeinen setzt man die N-Alkylsubstituierten Carbonsäureamide in Mengen zwischen 0,1 und 10 Mol-%, bezogen auf 1 Mol 5-Methyl-2,4-thiazolidindion, ein. Vorzugsweise arbeitet man mit Mengen zwischen 1 und 5 Mol-%.

Die Reaktionstemperaturen liegen im allgemeinen zwischen etwa 50°C und dem Siedepunkt des Reaktionsgemischs (etwa 105°C). Zur Erzielung einer möglichst raschen Umsetzung wird bevorzugt bei der Rückflusstemperatur des Reaktionsgemischs (100-110°C) gearbeitet.

Man erhitzt so lange, bis die HCl-Gasentwicklung praktisch beendet ist. Arbeitet man bei der Rückflusstemperatur des Reaktionsgemischs, so ist die Umsetzung im allgemeinen nach 5 bis 10 Stunden beendet.

Die Aufarbeitung des Reaktionsgemischs und Reinisolierung des 2,4-Dichlor-5-methylthiazols kann auf zwei verschiedenen Wegen in üblicher Weise erfolgen: Entweder gibt man das Reaktionsgemisch in überschüssiges Wasser und extrahiert nach Hydrolyse des überschüssigen Phosphoroxidchlorids das 2,4-Dichlor-5-methylthiazol mit einem organischen, mit Wasser nicht mischbaren Lösungsmittel, z.B. Methylenchlorid, und unterwirft die organische Phase anschliessend einer fraktionierten Destillation, oder man destilliert sowohl überschüssiges Phosphoroxidchlorid als auch das 2,4-Dichlor-5-methylthiazol aus dem Reaktionsgemisch ab, wobei gleichzeitig oder im Anschluss daran die beiden Verbindungen durch fraktionierte Destillation getrennt werden. Selbstverständlich kann man auch Mischformen der beiden geschilderten Aufarbeitungswege anwenden.

Das bei der Durchführung der Variante α) des erfindungsgemässen Verfahrens (b) benötigte 2,4-Dichlor-5-methylthiazol ist bisher noch nicht bekannt. Es lässt sich jedoch nach dem erfindungsgemässen Verfahren (a) herstellen.

Bei der Durchführung der Variante α) des erfindungsgemässen Verfahrens (b) wird 2,4-Dichlor-5-methylthiazol bei erhöhter Temperatur unter Belichtung mit Chlor umgesetzt. Im allgemeinen arbeitet man im Temperaturbereich zwischen 50°C und 250°C. Man kann mit oder ohne Verdünnungsmittel arbeiten. Als Verdünnungsmittel kommen alle unter den Reaktionsbedingungen inerten Lösungsmittel in Frage. Genannt seien perchlorierte organische Kohlenwasserstoffe wie Tetrachlormethan, Hexachlorbutadien, Octachlorcyclopenten oder anorganische Säurechloride wie Phosphoroxidchlorid.

Bei der Durchführung der Variante α) des erfindungsgemässen Verfahrens (b) kann man bezüglich der Chlorierungstechnik zwei verschiedene Wege einschlagen: Der erste Weg ($b_1$) ist von geringerer Selektivität und erfordert daher zur Reindarstellung der Chlorierungsprodukte (Ib), (Ic) und (Id) im allgemeinen eine anschliessende fraktionierte Destillation. Der zweite Weg ($b_2$) ist von höherer Selektivität und liefert die genannten Chlorierungsprodukte, insbesondere das 5-Chlormethyl-2,4-dichlorthiazol (Ib), im allgemeinen mit nur geringfügigen Beimengungen höher chlorierter Produkte.

Im einzelnen wird das Verfahren ($b_1$) so durchgeführt, dass man im Temperaturbereich zwischen 50 und 250°C unter Belichtung Chlorgas in eine Flüssigphase einleitet, die aus 2,4-Dichlor-5-methylthiazol und gegebenenfalls aus einem der vorgenannten chlorierungsfesten Verdünnungsmittel besteht. Im allgemeinen arbeitet man mit 1 bis 10 ml Verdünnungsmittel pro Gramm 2,4-Dichlor-5-methylthiazol. Als Lichtquelle setzt man im allgemeinen die üblichen UV-Lampen ein, indem man sowohl von aussen als auch von innen (Tauchlampe) bestrahlen kann. Wünscht man jedoch die Herstellung eines Chlorierungsgemisches, in dem die Verbindung mit dem niedrigsten Chlorierungsgrad, das 5-Chlormethyl-2,4-dichlorthiazol, überwiegt, so kann eine Lichtquelle mit geringerer Strahlungsenergie, z.B. eine Neonröhre (Abzugsbeleuchtung), von Vorteil sein.

Die Menge des anzuwendenden Chlors hängt einerseits davon ab, welches Chlorierungsprodukt man als Hauptprodukt anstrebt; naturgemäss bewirken höhere Chlormengen einen höheren Chlorierungsgrad des Reaktionsgemischs. Andererseits hängt die anzuwendende Chlormenge stark von den übrigen Reaktionsbedingungen, insbesondere Temperatur,

Lichtquelle und Apparatedimensionen ab. Im allgemeinen arbeitet man mit Chlormengen zwischen 1 und 15 Mol Chlor pro Mol 2,4-Dichlor-5-methylthiazol.

Die Reaktionszeiten hängen einerseits stark von der Reaktionstemperatur, andererseits von dem angestrebten Chlorierungsgrad des Reaktionsgemisches sowie von der Ansatzgrösse ab. Sie liegen daher im allgemeinen zwischen 0,5 und 100 Stunden.

Da die Siedepunkte von 5-Chlormethyl-2,4-dichlorthiazol (Ib) (Kp.: 118 bis 119°C/20 mbar) und von 2,4-Dichlor-5-(dichlormethyl)-thiazol (Ic) (Kp.: 120 bis 121°C/20 mbar) eng beieinander liegen, empfiehlt es sich zur Reindarstellung einer der beiden Verbindungen, die Chlorierung unter gaschromatographischer Verfolgung so zu steuern, dass im Falle der Reindarstellung von (Ib) Verbindung (Ic) noch nicht bzw. nur in geringfügigem Ausmass entstanden ist, während im Falle der Reindarstellung von (Ic) Verbindung (Ib) nicht mehr vorhanden ist.

Im Falle der Herstellung der Verbindung (Id) kann es zur Vermeidung der Bildung unerwünschter Nebenprodukte mit höherem Chlorgehalt von Vorteil sein, die Chlorierungsreaktion bei einem gaschromatographisch festgestellten Gehalt an (Id) von 50 bis 75% abzubrechen und das Gemisch aus (Ic) und (Id) durch fraktionierte Destillation zu trennen.

Eine geeignete Vorrichtung zur fraktionierten Destillation, beispielsweise eines Gemisches aus (Ic) und (Id), besteht z.B. in einer Vakuummantel-verspiegelten Füllkörperkolonne von 1 m wirksamer Länge, die mit Wilson-Glasspiralen von 3 bis 4 mm Durchmesser gefüllt ist und mit magnetischem Dampfteiler (Rücklaufverhältnis z.B. 80 : 2) versehen ist.

Das Chlorierungsverfahren ($b_2$) von höherer Selektivität, das mindestens zur Herstellung von 5-Chlormethyl-2,4-dichlorthiazol (Ib) geeignet ist, ist im Prinzip in der Deutschen Offenlegungsschrift 2 844 270 beschrieben.

Im einzelnen wird das Verfahren ($b_2$) z.B. zur Herstellung von 5-Chlormethyl-2,4-dichlorthiazol so durchgeführt, dass man das 2,4-Dichlor-5-methylthiazol mit Chlor unter Belichtung, insbesondere in Gegenwart von UV-Licht, umsetzt, indem man das 2,4-Dichlor-5-methylthiazol in einem Verdampfungsgefäss, auf das ein Rohr aufgesetzt ist, das im oberen Teil zu einem Kondensator und im unteren Teil zu einer Kolonne ausgebildet ist und in dessen Mitte sich der Reaktionsraum befindet, verdampft, den Dampf an dem Kondensator kondensiert, so dass das Kondensat auf eine in den Reaktionsraum eingelassene Vorrichtung tropft und dort mit dem durch die Vorrichtung eingeleiteten Chlor zu 5-Chlormethyl-2,4-dichlorthiazol reagiert, welches durch das von dem Kondensator tropfende Kondensat durch ein in die Kolonne eingelassenes Rohr in das Verdampfungsgefäss gespült wird.

Das Verfahren ($b_2$) wird bevorzugt bei der Siedetemperatur des 2,4-Dichlor-5-methylthiazols (ca. 200 bis 210°C) und ohne Verdünnungsmittel durchgeführt.

Durch die in der DE-OS 2 844 270 in ihrer Funktionsweise detailliert beschriebene Spezialapparatur wird bewirkt, dass z.B. aus 2,4-Dichlor-5-methylthiazol (Ia) in hoher Selektivität 5-Chlormethyl-2,4-dichlorthiazol (Ib) entsteht, das nur geringfügige Mengen höherchlorierte Produkte enthält. Die Bildung höherchlorierter Produkte kann man ausserdem dadurch unterdrücken, dass man z.B. das 2,4-Dichlor-5-methylthiazol nicht bis zu einem Umsatz von 100%, sondern nur bis zu einem Umsatz von z.B. 70 bis 90% chloriert.

Für die Varianten β) und γ) des Chlorierungsverfahrens (b) gilt sinngemäss das gleiche wie für die Variante α). Selbstverständlich kann man die Verbindungen mit mittlerem Chlorierungsgrad (Ib und Ic) einzeln oder als Gemische in Verbindungen mit höherem Chlorierungsgrad (Ic und Id) überführen.

Die Verbindungen der Formel (I) sind wertvolle Ausgangsstoffe zur Herstellung hochwirksamer Herbizide. Hierzu werden zunächst ein oder mehrere Chloratome gegen Fluor ausgetauscht, z.B.

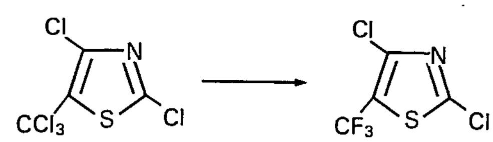

und die so erhaltenen Fluor-Verbindungen mit Hydroxyessigsäureamiden zu den herbizid hochwirksamen substituierten Thiazol-2-yl-oxyacetamiden umgesetzt, z.B.

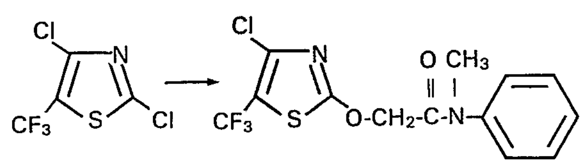

(vgl. z.B. DE-OS 2 914 003, DE-OS 3 004 326, EP-A-18 497 sowie Beispielteil).

Darüber hinaus sind die Verbindungen der Formel (I) selbst fungizid wirksam, insbesondere gegen Piricularia oryzae und gegen Xanthomonas oryzae im Reis.

A) *Herstellungbeispiele für Verbindungen der Formel (I)*

*Beispiel A-1*

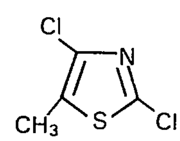

Eine Mischung aus 750 ml Phosphoroxidchlorid, 157,2 g (1,2 Mol) 5-Methyl-2,4-thiazolidindion und 4 ml Dimethylformamid wurde unter Rühren so lange unter Rückfluss eritzt, bis die Gasentwicklung praktisch beendet war (etwa 6 Stunden). Die erkaltete Reaktionsmischung wurde anschliessend unter gutem Rühren anteilweise auf 5 kg Eis ausgetragen. Dann wurde dreimal mit je etwa einem Liter Methylenchlorid ausgeschüttelt, das Methylenchlorid im Rotationsverdampfer (Rotavapor) abgezogen und der Rückstand (187,3 g) destilliert. Man erhielt bei 86°C/18 mbar 159,1 g (entsprechend 78,9% der

Theorie) 2,4-Dichlor-5-methylthiazol von einer gaschromatographischen Reinheit von 99,9%. Siedepunkt bei gewöhnlichem Druck: 203°C.

*Beispiel A-2*

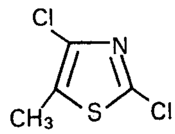

Es wurde analog Beispiel 1 verfahren mit dem Unterschied, dass statt 4 ml nur 2 ml Dimethylformamid eingesetzt wurden. Die Reinausbeute an 2,4-Dichlor-5-methylthiazol betrug 74,8% der Theorie.

*Beispiel A-3*

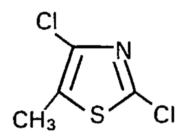

Es wurde analog Beispiel 1 verfahren mit dem Unterschied, dass statt 4 ml nur 2 ml Dimethylformamid und statt 750 ml nur 375 ml Phosphoroxidchlorid umgesetzt wurden. Die Reinausbeute an 2,4-Dichlor-5-methylthiazol betrug 66,6% der Theorie.

*Beispiel A-4*

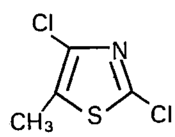

Zunächst wurde analog Beispiel 1 verfahren. Nach beendeter Gasentwicklung wurden dann im Wasserstrahlvakuum bei etwa 20 bis 86°C/18 mbar bis zu einer Heizbadtemperatur von etwa 150°C alle destillierbaren Anteile der Reaktionsmischung abdestilliert. Anschliessend wurde die Hauptmenge des wiedergewonnenen Phosphoroxidchlorids an einer Kolonne bei gewöhnlichem Druck bei etwa 106°C abdestilliert. Der Rückstand wurde in 2 l Eiswasser verrührt, die wässrige Phase nach Hydrolyse der Restmengen Phosphoroxidchlorid dreimal mit je einem Liter Methylenchlorid ausgeschüttelt, das Methylenchlorid im Rotavapor abgezogen und der Rückstand (152,4 g) destilliert. Man erhielt bei 83 bis 85°C/16 mbar 147,7 g (entsprechend 73,3% der Theorie) gaschromatographisch reines 2,4-Dichlor-5-methylthiazol.

*Beispiel A-5*

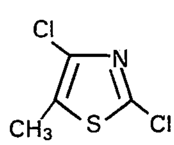

Eine Mischung aus 3 Liter Phosphoroxidchlorid, 630 g (4,81 Mol) 5-Methyl-2,4-thiazolidindion und 16 ml Dimethylformamid wurde unter Rühren so lange unter Rückfluss erhitzt, bis die Gasentwicklung praktisch beendet war (etwa 8 Stunden). Danach wurden im Wasserstrahlvakuum bis zu einer Heizbadtemperatur von etwa 180°C alle destillierbaren Anteile der Reaktionsmischung abdestilliert. Das Destillat bestand nach der gaschromatographischen Analyse zu 99,7% aus Phosphoroxidchlorid und aus 2,4-Dichlor-5-methylthiazol. Der gaschromatographisch ermittelte Gehalt an 2,4-Dichlor-5-methylthiazol betrug 595,7 g (entsprechend 73,7% der Theorie). Anschliessende fraktionierte Destillation an einer Kolonne mit Rücklaufteiler lieferte bei 86°C/19 mbar reines 2,4-Dichlor-5-methylthiazol.

*Beispiel A-6*

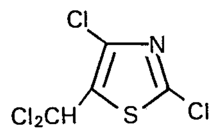

+

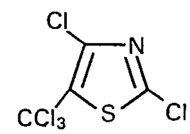

In einem zylindrischen Gefäss, das mit Thermometer, Rückflusskühler, einer Labortauchlampe (Hg-Hochdruck-Strahler Hanau) und am Boden mit einer Glasfritte für Gaseinlass versehen ist, wurden in eine Mischung aus 336 g (2 Mol) 2,4-Dichlor-5-methylthiazol und 350 ml Tetrachlormethan im Verlauf von 15 Stunden 1690 g (23,8 Mol) Chlor eingeleitet. In den ersten 10 Stunden der Chlorierungszeit betrug die Sumpftemperatur 80 bis 85°C; in den letzten 5 Stunden stieg die Temperatur (durch Verdampfungsverluste von $CCl_4$) bis auf etwa 120°C an.

Nach dem GC bestand das Reaktionsgemisch zu 39,1% aus 5-Dichlormethyl-2,4-dichlorthiazol und zu 53,5% aus 5-Trichlormethyl-2,4-dichlorthiazol. Der Rest bestand aus unerwünschten Nebenprodukten mit höherem Chlorgehalt. Destillative Aufarbeitung siehe nächstes Beispiel Nr. 7.

*Beispiel A-7*

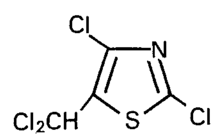

+

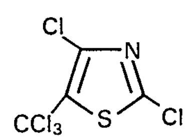

In einer Apparatur analog Beispiel 6 wurden bei einer Badtemperatur von 100°C und einer Sumpftemperatur zwischen 80 und 100°C in eine Mischung aus 1008 g (6 Mol) 2,4-Dichlor-5-methylthiazol und 1050 ml Tetrachlormethan im Verlauf von etwa 62 Stunden 5170 g (72,8 Mol) Chlor eingeleitet.

Nach dem GC bestand das Reaktionsgemisch zu 28,8% aus 5-Dichlormethyl-2,4-dichlorthiazol und zu 64,7% aus 5-Trichlormethyl-2,4-dichlorthiazol. Der Rest bestand aus unerwünschten Nebenprodukten mit höherem Chlorgehalt. Die destillative Aufarbeitung erfolgte gemeinsam mit dem Produktgemisch des vorigen Beispiels Nr. 6. Hierfür wurde eine Vakuummantel-verspiegelte Füllkörperkolonne

(Inhalt: Wilson-Glasspiralen von 3 bis 4 mm Durchmesser) von 1 m wirksamer Länge verwendet, die mit einem Kolonnenkopf mit magnetischem Dampfteiler (Rücklaufverhältnis 80 : 2) versehen war.

Man erhielt bei 120 bis 121°C/20 mbar 538 g (27,9%) 5-Dichlormethyl-2,4-dichlorthiazol (GC-Reinheit: 98,3%) und bei 136 bis 137°C/19 mbar 1301 g (58,6%) 5-Trichlormethyl-2,4-dichlorthiazol (GC-Reinheit: 97,8%).

*Beispiel A-8*

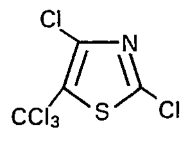

In einer Apparatur analog Beispiel 6 wurden in 1008 g (6 Mol) 2,4-Dichlor-5-methylthiazol bei 200 bis 210°C im Verlauf von 6 Stunden 340 g Chlor und anschliessend zwischen 210 und 240°C im Verlauf von 7 Stunden weitere 860 g Chlor (insgesamt 1200 g = 16,9 Mol) eingeleitet. Das Gaschromatogramm des Rohgemischs zeigte an, dass von den vier möglichen 2,4-Dichlorthiazolen (Ia) bis (Id) nur 2,4-Dichlor-5-(trichlormethyl)-thiazol (Id) vorhanden war. Die destillative Aufarbeitung lieferte bei 136 bis 137°C/19 mbar 773 g (49,1%, bezogen auf die eingesetzte Chlormenge) 2,4-Dichlor-5-(trichlormethyl)-thiazol, GC-Reinheit: 97,1%.

*Beispiel A-9*

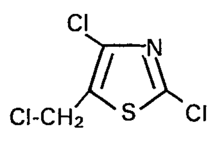

205 g (1,22 Mol) 2,4-Dichlor-5-methylthiazol wurden in einer Apparatur, die in der DT-OS 2 844 270 auf den Seiten 18 und 23 beschrieben ist, unter UV-Bestrahlung mit einem Hg-Hochdruck-Strahler unter Sieden (Sumpftemperatur anfangs 205°C) chloriert. Sobald die Sumpftemperatur 235°C erreicht hatte, wurde die Chlorierung abgebrochen: Umsatz etwa 80%. Das Chlorierungsgemisch wies nach der gaschromatographischen Analyse die folgende Zusammensetzung auf:

19,5% 2,4-Dichlor-5-methylthiazol

79,0% 5-Chlormethyl-2,4-dichlorthiazol

1,5% 2,4-Dichlor-5-(dichlormethyl)-thiazol.

Die fraktionierte Destillation an einer Füllkörperkolonne von 30 cm wirksamer Länge, gefüllt mit Glasringen von 2 mm Durchmesser und 2 mm Länge, lieferte beim Kp. 118-119°C/20 mbar 145 g (72%, bezogen auf den Umsatz) 5-Chlormethyl-2,4-dichlorthiazol; $n_D^{20}$ = 1,5835; GC-Reinheit: 98,5%.

*Beispiel A-10*

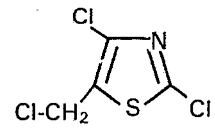

In einem Dreihalskolben, der mit Rührer, Thermometer, Rückflusskühler und Gaseinleitungsrohr versehen war, wurden in eine Mischung aus 117,5 g (0,7 Mol) 2,4-Dichlor-5-methylthiazol und 400 ml Tetrachlormethan bei Rückflusstemperatur (etwa 82°C) im Verlauf von 8 Stunden 100 g (1,41 Mol) Chlor eingeleitet. Als Lichtquelle diente eine in einem Abstand von etwa 80 cm befindliche Neon-Tageslicht-Röhre (Abzugsbeleuchtung). Das Chlorierungsgemisch wies nach der gaschromatographischen Analyse (Eliminierung von Tetrachlormethan) die folgende Zusammensetzung auf:

16,0% 2,4-Dichlor-5-methylthiazol
73,6% 5-Chlormethyl-2,4-dichlorthiazol
10,0% 2,4-Dichlor-5-(dichlormethyl)-thiazol.

*Beispiel A-11*

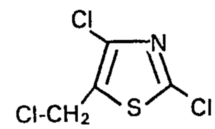

In einer Apparatur analog Beispiel 10 wurden in eine Mischung aus 78,9 g (0,47 Mol) 2,4-Dichlor-5-methylthiazol und 200 ml Tetrachlormethan bei Rückflusstemperatur im Verlauf von 6 Stunden 100 g (1,41 Mol) Chlor eingeleitet. Als einzige Lichtquelle diente diffuses Tageslicht (bedeckter Himmel, Abstand zum Fenster ca. 5 m). Das Chlorierungsgemisch wies nach der gaschromatographischen Analyse die folgende Zusammensetzung auf:

89,3% 2,4-Dichlor-5-methylthiazol
9,6% 5-Chlormethyl-2,4-dichlorthiazol
1,0% 2,4-Dichlor-5-(dichlormethyl)-thiazol.

*Beispiel A-12*

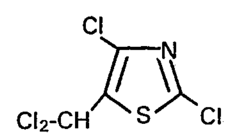

In einer Apparatur analog Beispiel 6 wurden in eine Mischung aus 59 g (0,35 Mol) 2,4-Dichlor-5-methylthiazol und 450 ml Tetrachlormethan im Verlauf von 6 Stunden bei der Rückflusstemperatur (ca. 80°C) 100 g (1,41 Mol) Chlor eingeleitet.

Nach dem GC bestand das Reaktionsgemisch aus:

2,0% 5-Chlormethyl-2,4-dichlorthiazol
89,4% 2,4-Dichlor-5-(dichlormethyl)-thiazol
7,9% 2,4-Dichlor-5-(trichlormethyl)-thiazol.

Durch fraktionierte Destillation an einer Füllkörperkolonne von etwa 30 cm Länge erhielt man als Hauptlauf bei Kp. 122-125°C/20 mbar 48,5 g 2,4-Dichlor-5-(dichlormethyl)-thiazol in einer GC-Reinheit von 94,0%. Ausbeute (bezogen auf 100%iges Produkt): 54,8% der Theorie.

*Beispiel A-13*

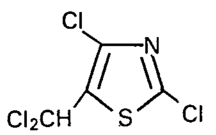

+

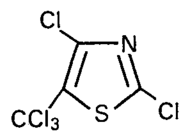

In einer Apparatur analog Beispiel A-6 wurden in eine Mischung aus 336 g (2 Mol) 2,4-Dichlor-5-methylthiazol und 500 ml Tetrachlormethan bei der Rückflusstemperatur (ca. 80°C) im Verlauf von 10 Stunden 1000 g (14,1 Mol) Chlor eingeleitet. Nach dem GC bestand das Reaktionsgemisch zu 71,6% aus 2,4-Dichlor-5-(dichlormethyl)-thiazol, zu 24,5% aus 2,4-Dichlor-5-(trichlormethyl)-thiazol und zu 2,2% aus unerwünschten Nebenprodukten mit höherem Chlorgehalt.

*Beispiel A-14*

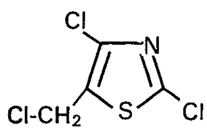

In einem Dreihalskolben, der mit Rührer, Thermometer, Rückflusskühler und Gaseinleitungsrohr versehen war, wurden in eine Mischung aus 336 g (2 Mol) 2,4-Dichlor-5-methylthiazol und 500 ml Tetrachlormethan bei Rückflusstemperatur (ca. 85°C) im Verlauf von 4,5 Stunden 140 g (1,97 Mol) Chlor eingeleitet. Als Lichtquelle diente eine in einem Abstand von etwa 80 cm befindliche Neon-Tageslicht-Röhre (Abzugsbeleuchtung). Das Chlorierungsgemisch wies nach der GC-Analyse (Eliminierung von Tetrachlormethan) die folgende Zusammensetzung auf:

91,4% 2,4-Dichlor-5-methylthiazol
8,5% 5-Chlormethyl-2,4-dichlorthiazol
0,1% 2,4-Dichlor-5-(dichlormethyl)-thiazol.

*Beispiel A-15*

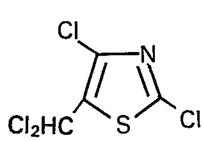

+

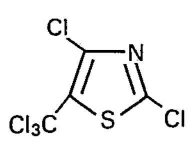

In einer Apparatur analog Beispiel 6 wurden in 2167 g eines Gemisches aus 72,4% 2,4-Dichlor-5-methylthiazol, 9,1% 5-Chlormethyl-2,4-dichlorthiazol und 18,4% 2,4-Dichlor-5-dichlormethylthiazol bei einer Innentemperatur von 140 bis 160°C im Verlauf von 12 Stunden 4290 g (60,4 Mol) Chlor eingeleitet. Das Gaschromatogramm des Rohgemischs erbrachte folgende Zusammensetzung: 18,1% 2,4-Dichlor-5-dichlormethylthiazol und 80,9% 2,4-Dichlor-5-trichlormethylthiazol.

B) *Herstellungsbeispiele für fluorhaltige Zwischenprodukte:*

*Beispiele B-1*

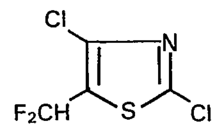

430 g (1,8 Mol) 2,4-Dichlor-5-dichlormethyl-1,3-thiazol werden mit 650 ml wasserfreier Flusssäure im VA-Autoklaven bei 137 - 140°C/18 - 22 bar fluoriert. Der entstehende Chlorwasserstoff wird laufend entspannt. Nach Ende der Reaktion wird die überschüssige Flusssäure im Vakuum bei Raumtemperatur abgezogen, der Rückstand wird auf Eiswasser gegeben, in Dichlormethan aufgenommen, über Natriumsulfat getrocknet und destilliert.

Man erhält 275 g (74,3% der Theorie) 2,4-Dichlor-5-difluormethyl-1,3-thiazol, Kp. 12 mbar/65 - 66°C; $n_D^{20}$ = 1,5070; sowie 30 g höher fluorierte Anteile.

*Beispiele B-2 und B-3*

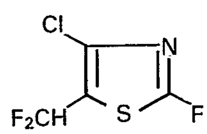

und

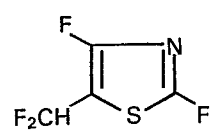

230 g (1,12 Mol) 2,4-Dichlor-5-difluormethyl-1,3-thiazol werden mit 131 g (2,25 Mol) Kaliumfluorid in 339 ml Tetramethylensulfon 3 Stunden bei 160°C gerührt. Anschliessend wird das fluorierte Produkt bis zum Siedepunkt des Tetramethylensulfons im Vakuum abdestilliert.

Durch Redestillation erhält man:

76 g (40% der Theorie) 2,4-Difluor-5-difluormethyl-1,3-thiazol vom Siedepunkt Kp.: 108-9°C; Brechungsindex $n_D^{20}$ = 1,4108

und

47 g (22,4% der Theorie) 2-Fluor-4-chlor-5-difluormethyl-1,3-thiazol vom Siedepunkt Kp.: 141-3°C; Brechungsindex $n_D^{20}$ = 1,4528

sowie

40 g Ausgangsverbindung.

*Beispiel B-4 und B-5*

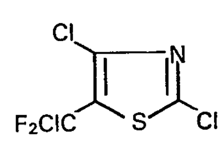

und

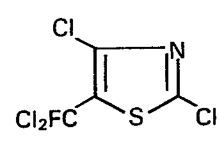

227 g (0,836 Mol) 2,4-Dichlor-5-trichlormethyl-1,3-thiazol werden mit 200 ml wasserfreier Flusssäure im VA-Autoklaven bei 50°C/3-8 bar fluoriert. Der entstehende Chlorwasserstoff wird laufend entspannt. Nach Ende der Reaktion (ca. 4 Stunden) wird auf Raumtemperatur abgekühlt und die überschüssige Flusssäure im Vakuum bis 100 mbar abgezogen. Der Rückstand wird auf Eiswasser gegeben, in Dichlormethan aufgenommen, über Natriumsulfat getrocknet und destilliert.

Man erhält:

84 g (39,6% der Theorie) 2,4-Dichlor-5-difluorchlormethyl-1,3-thiazol vom Siedepunkt Kp.: 76-8°C/18 mbar, Brechungsindex $n_D^{20}$ = 1,5120

und

67 g (33,7% der Theorie) 2,4-Dichlor-5-dichlorfluormethyl-1,3-thiazol vom Siedepunkt Kp.: 105-107°C/18 mbar, Brechungsindex $n_D^{20}$ = 1,5539

sowie

12 g Ausgangsverbindung.

Wird die Reaktion bei 60°C/5 bar durchgeführt, erhält man mit einer Ausbeute von 71% der Theorie 2,4-Dichlor-5-difluorchlormethyl-1,3-thiazol.

*Beispiel B-6*

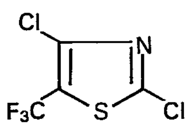

500 g (1,84 Mol) 2,4-Dichlor-5-trichlormethyl-1,3-thiazol werden mit 740 ml wasserfreier Flusssäure im VA-Autoklaven bei 120 - 140°C/25 - 20 bar 3 Stunden fluoriert. Der entstehende Chlorwasserstoff wird laufend entspannt. Nach Ende der Reaktion wird abgekühlt und die überschüssige Flusssäure im Wasserstrahlvakuum bis 100 mbar abgezogen. Der Rückstand wird auf Eiswasser gegeben, in Methylenchlorid aufgenommen, über Natriumsulfat getrocknet und destilliert.

Man erhält:

280 g (68,5% der Theorie) 2,4-Dichlor-5-trifluormethyl-1,3-thiazol vom Siedepunkt Kp.: 50°C/ 16 mbar; Brechungsindex $n_D^{20}$ = 1,4710

sowie

63 g anfluorierte Verbindungen.

C) *Herstellungsbeispiele für herbizid wirksame Thiazol-2-yl-oxyacetamide*

*Beispiel C-1*

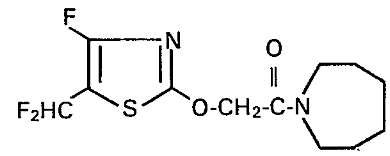

Zu 7,9 g (0,05 Mol) Hydroxyessigsäure-N,N-hexamethylenamid und 3,1 g (0,05 Mol) Kaliumhydroxid in 100 ml Isopropanol tropft man bei −20°C langsam unter Rühren 8,5 g (0,05 Mol) 2-Chlor-4-fluor-5-difluormethyl-1,3-thiazol in 10 ml Acetonitril und rührt nach beendeter Zugabe für weitere 12 Stunden bei −20°C. Wenn sich im Dünnschichtchromatogramm kein Ausgangsprodukt mehr nachweisen lässt, giesst man die Reaktionsmischung in Wasser, saugt das kristalline Produkt ab und wäscht mit Wasser und wenig kaltem Ligroin nach. Man erhält 12 g (80% der Theorie) an 2-(4-Fluor-5-difluormethyl-thiazol-2-yloxy)-essigsäure-N,N-hexamethylenamid vom Schmelzpunkt 66°C.

In entsprechender Weise erhält man die folgenden Verbindungen der folgenden allgemeinen Formel – vgl. Tabelle 1:

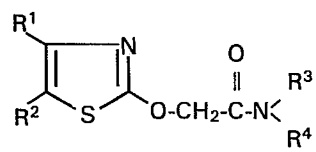

(Fallen die Substanzen als Öle an, werden sie, wie allgemein üblich, durch Extraktion mit einem organischen Lösungsmittel aus der wässrigen Mischung isoliert).

Tabelle 1

| Beispiel Nr. | $R^1$ | $R^2$ | $-N\langle^{R^3}_{R^4}$ | physikalische Daten |
|---|---|---|---|---|
| C-2 | Cl | $CH_3$ | 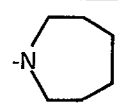 | Fp: 61°C |
| C-3 | Cl | $CH_3$ | $-N\langle^{C_2H_5}_{C_2H_5}$ | Fp: 42°C |
| C-4 | Cl | $CH_3$ | $-N\langle^{CH_3}_{C_6H_5}$ | Fp: 82°C |

Tabelle 1 (Fortsetzung)

| Beispiel Nr. | $R^1$ | $R^2$ | $R^3$<br>$-N<$<br>$R^4$ | physikalische Daten |
|---|---|---|---|---|
| C-5 | Cl | $CHCl_2$ | $CH_3$<br>$-N<$<br>$C_6H_5$ | Fp: 94°C |
| C-6 | Cl | $CF_2Cl$ | $CH_3$<br>$-N<$<br>$C_6H_5$ | Fp: 84°C |
| C-7 | Cl | $CF_2Cl$ | $C_2H_5$<br>$-N<$<br>$C_2H_5$ | $n_D^{20}$ = 1,5060 |
| C-8 | Cl | $CF_2Cl$ | $OCH_3$<br>$-N<$<br>$CH-C_2H_5$<br>\|<br>$CH_3$ | $n_D^{20}$ = 1,4950 |
| C-9 | Cl | $CHF_2$ | $O-CH_2CH_2OC_2H_5$<br>$-N<$<br>$CH(CH_3)_2$ | $n_D^{20}$ = 1,4891 |
| C-10 | Cl | $CHF_2$ | $C_2H_5$<br>$-N<$<br>$C_2H_5$ | Fp: 64°C |
| C-11 | Cl | $CHF_2$ | 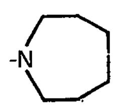 | Fp: 72°C |
| C-12 | Cl | $CHF_2$ | $CH_3$<br>$-N<$<br>$C_6H_5$ | Fp: 78°C |
| C-13 | F | $CHF_2$ | $C_2H_5$<br>$-N<$<br>$C_2H_5$ | Fp: 50°C |
| C-14 | Cl | $CHF_2$ | $CH_3$<br>$-N<$<br>$C_6H_5$ | Fp: 74°C |
| C-15 | Cl | $CF_3$ | $CH_3$<br>$-N<$<br>$C_6H_5$ | Fp: 115°C |
| C-16 | Cl | $CF_3$ | 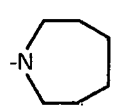 | Fp: 70°C |
| C-17 | Cl | $CF_3$ | $C_2H_5$<br>$-N<$<br>$C_2H_5$ | Fp: 39°C |
| C-18 | Cl | $CF_3$ | $CH_2CH=CH_2$<br>$-N<$<br>$CH_2CH=CH_2$ | Fp: 66°C |

Tabelle 1 (Fortsetzung)

| Beispiel Nr. | $R^1$ | $R^2$ | $R^3$<br>$-N<$<br>$R^4$ | physikalische Daten |
|---|---|---|---|---|
| C-19 | Cl | $CF_3$ | $CH_3$<br>$-N<$<br>$CH_3$ | Fp: 58°C |
| C-20 | Cl | $CF_3$ | 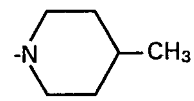 | Fp: 72°C |
| C-21 | Cl | $CF_3$ | 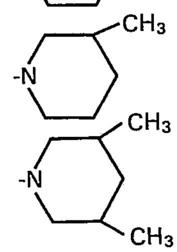 | $n_D^{20}$ = 1,4908 |
| C-22 | Cl | $CF_3$ | | $n_D^{20}$ = 1,4965 |
| C-23 | Cl | $CF_3$ | $(CH_2)_2-CH_3$<br>$-N<$<br>$(CH_2)_2-CH_3$ | $n_D^{20}$ = 1,4774 |
| C-24 | Cl | $CF_3$ | $OCH_3$<br>$-N<$<br>$CHC_2H_5$<br>I<br>$CH_3$ | $n_D^{20}$ = 1,4715 |
| C-25 | Cl | $CF_3$ | $O-CH_2CH_2OC_2H_5$<br>$-N<$<br>$CH(CH_3)_2$ | $n_D^{20}$ = 1,5664 |
| C-26 | F | $CHF_2$ | $OCH_2CH_2OC_2H_5$<br>$-N<$<br>$CH(CH_3)_2$ | Fp: 54-56°C |
| C-27 | F | $CHF_2$ | $OCH_3$<br>$-N<$<br>$CH-C_2H_5$<br>I<br>$CH_3$ | $n_D^{20}$ = 1,4715 |

*Verwendungsbeispiele*

*Beispiel I*

Pre-emergence-Test

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Eine deutliche Überlegenheit in der herbiziden Wirksamkeit bei vergleichbarer Nutzpflanzenselektivität gegenüber dem Stand der Technik zeigen bei diesem Test z.B. die Verbindungen gemäss folgender Herstellungsbeispiele: C-12, C-15, C-16, C-17 und C-24.

*Beispiel II*

Xanthomonas oryzae-Test/Bakteriose/Reis systemisch

Lösungsmittel: 122,5 Gewichtsteile Dimethylformamid

Emulgator: 2,5 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften werden 100 ml der Wirkstoffzubereitung auf Einheitserde gegossen, in der junge Pflanzen angezogen wurden. 3 Tage nach der Behandlung werden die Pflanzen mit einer wässrigen Suspension von Xanthomonas oryzae durch Stichverletzung inokuliert. Danach verbleiben die Pflanzen 10 bis 14 Tage bis zur Auswertung in einem Gewächshaus bei 24 bis 26 °C und 70 bis 80% rel. Luftfeuchtigkeit.

Eine deutliche biologische Wirkung zeigt bei diesem Test z.B. die Verbindung (Ic).

Tabelle A

Xanthomonas oryzae-Test/Bakteriose/Reis systemisch

| Wirkstoffe | Aufwandmenge in mg Wirkstoff pro 100 $cm^2$ | Krankheitsbefall in % der unbehandelten Kontrolle systemisch |
|---|---|---|
| 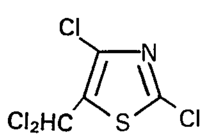 (Ic) | 10 | 20 |

**Patentansprüche**

1. 2,4-Dichlorthiazol-Derivate der Formel

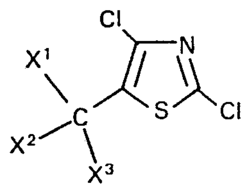

(I)

in welcher
$X^1$ für Wasserstoff oder Chlor,
$X^2$ für Wasserstoff oder Chlor und
$X^3$ für Wasserstoff oder Chlor steht.

2. Verfahren zur Herstellung von 2,4-Dichlorthiazol-Derivaten der Formel (I) gemäss Anspruch 1, dadurch gekennzeichnet, dass man

a) 5-Methyl-2,4-thiazolidindion der Formel

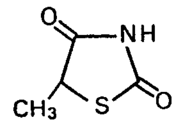

(II)

mit mindestens 2 Mol Phosphoroxidchlorid pro Mol an 5-Methyl-2,4-thiazolidindion in Gegenwart katalytischer Mengen N-Alkyl-substituierter Carbonsäureamide umsetzt, wobei man 2,4-Dichlor-5-methylthiazol der Formel (Ia) erhält,
oder dass man

b) α) 2,4-Dichlor-5-methylthiazol der Formel

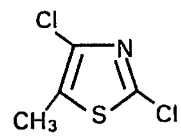

(Ia)

oder

β) 5-Chlormethyl-2,4-dichlorthiazol der Formel

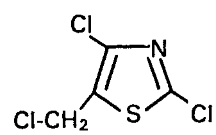

(Ib)

oder

γ) 2,4-Dichlor-5-(dichlormethyl)-thiazol der Formel

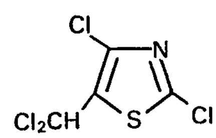

(Ic)

bei erhöhter Temperatur unter Belichtung mit Chlor umsetzt.

**Claims**

1. 2,4-Dichlorothiazole derivatives of the formula

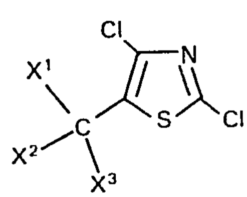

(I)

in which
$X^1$ stands for hydrogen or chlorine,
$X^2$ stands for hydrogen or chlorine and
$X^3$ stands for hydrogen or chlorine.

2. Process for the preparation of 2,4-dichlorothiazole derivatives of the formula (I) according to claim 1, characterized in that

a) 5-methyl-2,4-thiazolidinedione of the formula

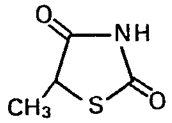

(II)

is reacted with at least 2 moles of phosphorus oxychloride per mole of 5-methyl-2,4-thiazolidinedione in the presence of catalytic amounts of N-alkylsubstituted carboxylic acid amides, 2,4-dichloro-5-methylthiazole of the formula (Ia) being obtained, or in that

b) α) 2,4-dichloro-5-methylthiazole of the formula

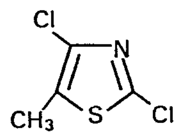

(Ia)

or

β) 5-chloromethyl-2,4-dichlorothiazole of the formula

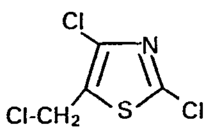

(Ib)

or

γ) 2,4-dichloro-5-(dichloromethyl)-thiazole of the formula

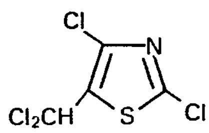

(Ic)

is reacted with chlorine at elevated temperature under exposure to light.

## Revendications

1. Dérivés du 2,4-dichlorthiazole, de formule

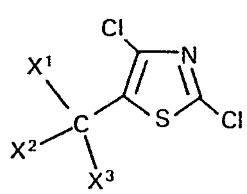

(I)

dans laquelle
$X^1$ représente un atome d'hydrogène ou de chlore,
$X^2$ représente un atome d'hydrogène ou de chlore, et
$X^3$ représente un atome d'hydrogène ou de chlore.

2. Procédé pour préparer des dérivés de 2,4-dichlorothiazole de formule (I) selon la revendication 1, caractérisé en ce que:

a) on fait réagir la 5-méthyl-2,4-thiazolidinedione de formule

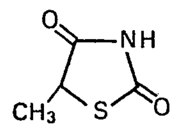

(II)

avec au moins 2 moles d'oxychlorure de phosphore par mole de 5-méthyl-2,4-thiazolidinedione, en présence de quantités catalytiques d'amides d'acides carboxyliques à substituants alkyles sur l'azote, de sorte que l'on obtient le 2,4-dichloro-5-methylthiazole de formule (Ia),
ou

b) on fait réagir α) le 2,4-dichloro-5-méthylthiazole de formule

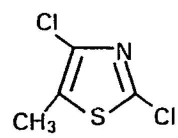

(Ia)

ou

β) le 5-chlorométhyl-2,4-dichlorothiazole de formule

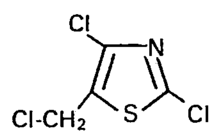

(Ib)

ou

γ) le 2,4-dichloro-5-(dichlorométhyl)-thiazole de formule

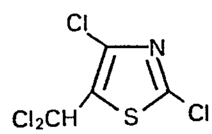

(Ic)

à température élevée, avec irradiation par éclairage, avec du chlore.